# EUROPEAN PATENT APPLICATION

(11) **EP 4 537 815 A1**
(43) Date of publication of application: **16.04.2025**
(21) Application number: 23306748.7
(22) Date of filing: 10.10.2023
(51) Int. Cl.: A61K 9/107, A61K 45/06, A61P 1/00, A61P 31/04, A61K 38/00

(54) **COMPOSITION COMPRISING A LASSO PEPTIDE FORMULATED IN A PHARMACEUTICALLY ACCEPTABLE NANOEMULSION**

(71) Applicant: Muséum National D'histoire Naturelle, 75005 Paris (FR); Centre National de la Recherche Scientifique, 75016 Paris (FR); Université de Caen Normandie, 14032 Caen Cedex 5 (FR)
(72) Inventor: LI, Yanyan, 75005 Paris (FR); GOULARD, Christophe, 75005 Paris (FR); HARTKE, Axel, 14032 Caen Cedex 5 (FR); GIRAUD, Caroline, 14032 Caen Cedex 5 (FR); BOUDRIOUA, Abdelhakim, 14032 Caen Cedex 5 (FR); BAËTZ, Benjamin, 14032 Caen Cedex 5 (FR); DESMADRIL, Solenn, 14032 Caen Cedex 5 (FR); GROO, Anne-Claire, 14032 Caen Cedex (FR); MALZERT FREON, Aurélie, 14032 Caen Cedex (FR)
(74) Representative: Gevers & Orès

(57) **Abstract**

The present invention relates to a lasso peptide, and a composition comprising a lasso peptide formulated in a pharmaceutically acceptable nanoemulsion and an antibiotic, to said composition for use as a medicine, and to a method for producing said composition. The composition of the present invention comprises a lasso peptide formulated in a pharmaceutically acceptable nanoemulsion and an antibiotic, wherein the composition is obtained by a process comprising a step of adding said antibiotic to a pharmaceutically acceptable nanoemulsion already comprising the lasso peptide or during the step of forming the nanoemulsion comprising the lasso peptide. The composition of the present invention finds an application for example for the treatment of an infection by a Gram-positive bacterium.

## Description

### Technical field of the invention

The present invention relates to a composition comprising a lasso peptide formulated in a pharmaceutically acceptable nanoemulsion and an antibiotic, to said composition for use as a medicine, and to a method for producing said composition.

The composition of the present invention finds an application for example for the treatment of an infection by a Gram-positive bacterium.

### Background of the invention

The number of infections caused by multi-drug resistant bacteria is increasing worldwide. *Enterococcus faecalis, Enterococcus faecium, Staphylococcus aureus* and *Clostridioides difficile* are Gram-positive bacteria that can cause severe nosocomial infections.

Cases have been reported in which these pathogenic bacteria have developed resistance against vancomycin, a glycopeptide antibiotic. As a result, treating an infection caused by a vancomycin-resistant strain of these bacteria becomes more complicated.

World Health Organization published in 2017 on its website the article "WHO publishes list of bacteria for which new antibiotics are urgently needed" (Ref. No. 1). This article presents a "list of bacteria for which new antibiotics are urgently needed". In this list, both *Enterococcus faecium* and *Staphylococcus aureus* appear in the high priority category, with a mention of their resistance to vancomycin.

Cases of *Staphylococcus aureus, Enterococcus faecalis* and *Enterococcus faecium* exhibiting resistance to teicoplanin, another glycopeptide antibiotic, have also been reported in documents McCallum N, Karauzum H, Getzmann R, Bischoff M, Majcherczyk P, Berger-Bächi B, Landmann R. In vivo survival of teicoplanin-resistant Staphylococcus aureus and fitness cost of teicoplanin resistance. Antimicrob Agents Chemother. 2006 Jul;50(7):2352-60 (Ref. No. 2) and Kawalec M, Gniadkowski M, Kedzierska J, Skotnicki A, Fiett J, Hryniewicz W. Selection of a teicoplanin-resistant Enterococcus faecium mutant during an outbreak caused by vancomycin-resistant enterococci with the VanB phenotype. J Clin Microbiol. 2001 Dec;39(12):4274-82 (Ref. No. 3).

To date, the need remains for pharmaceutical compositions suitable to efficiently treat infections caused by a bacterium exhibiting one of the above-listed resistance to antibiotics.

### Description of the invention

To fulfill the above-described need the inventors have precisely developed a composition comprising a lasso peptide and an antibiotic that meets the above expressed needs. They also developed a method to produce said composition as well as uses of said composition as a medicine.

### Compositions:

Particularly, the present disclosure relates to a composition comprising a lasso peptide formulated in a pharmaceutically acceptable nanoemulsion and an antibiotic, wherein the composition is obtained by a process comprising a step of adding said antibiotic to a pharmaceutically acceptable nanoemulsion already comprising the lasso peptide or during the preparation of a pharmaceutically acceptable nanoemulsion of said lasso peptide.

The composition according to the present disclosure comprises a lasso peptide. A lasso peptide may be defined as a macrocyclized peptide displaying a knotted topology.

Preferentially, the lasso peptide comprised in the composition according to the present disclosure is a lasso peptide produced by prokaryotes. Such lasso peptides are preferentially composed of 13-33 amino acids. Preferentially, they contain a 7- to 9-residue macrocycle at the N-terminus, through which a C-terminal tail is threaded, hence forming an interlocked structure, as disclosed in document Hegemann JD, Zimmermann M, Xie X, Marahiel MA. Lasso peptides: an intriguing class of bacterial natural products. Acc Chem Res. 2015 Jul 21;48(7):1909-19. (Ref. No. 4).

Preferentially, the lasso peptide comprised in the composition according to the present disclosure is a type I lasso peptide. Type I lasso peptides are lasso peptides which contain two disulfide bridges. Preferentially, the type I lasso peptides comprised in the composition according to the present disclosure contain two disulfide bridges that are formed between Cys1 and Cys13, Cys7 and Cys19, respectively.

Preferentially, the lasso peptide comprised in the composition according to the present disclosure is selected from the group consisting of siamycin I (also known as MS-271, CAS number: 164802-68-0, corresponding to SEQ ID No. 1), sviceucin (corresponding to SEQ ID No. 2), arcumycin (corresponding to SEQ ID No. 3), variants of siamycin I, variants of sviceucin, and variants of arcumycin.

**Table No. 1: Sequences of the cited lasso peptides**

| **Sequence identifier** | **Lasso peptide** | **Amino acid sequence** |
|---|---|---|
| SEQ ID No. 1 | Siamycin I | CLGVGSCNDFAGCGYAIVCFW |
| SEQ ID No. 2 | Sviceucin | CVWGGDCTDFLGCGTAWICV |
| SEQ ID No. 3 | Arcumycin | CLPSGDCPDFLGCGRAIWCI |
| SEQ ID No. 4 | Siamycin II | CLGIGSCNDFAGCGYAIVCFW |
| SEQ ID No. 5 | Aborycin | CLGIGSCNOFAGCGY AWCFW |
| SEQ ID No. 6 | Specialicin | CLGVGSCVDFAGCGYAWCFW |
| SEQ ID No. 7 | Humidimycin | CLGIGSCDDFAGCGYAIVCFW |

Siamycin I and sviceucin are two lasso peptides of which the chemical structures have been previously studied, as disclosed in document Li Y, Ducasse R, Zirah S, Blond A, Goulard C, Lescop E, Giraud C, Hartke A, Guittet E, Pernodet JL, Rebuffat S. Characterization of Sviceucin from Streptomyces Provides Insight into Enzyme Exchangeability and Disulfide Bond Formation in Lasso Peptides. ACS Chem Biol. 2015 Nov 20; 10(11):2641-9 (Ref. No. 5). Preferentially, the lasso peptide comprised in the composition according to the present disclosure is siamycin I.

By "variant" of the lasso peptides, we mean here another version of the peptides of siamycin I, sviceucin, or arcumycin, having the same or substantially the same activity as siamycin I, sviceucin, or arcumycin, respectively. For example, the variant may have additional or alternative amino acids, while keeping the same or substantially the same activity as siamycin I, sviceucin, or arcumycin, respectively.

Preferentially, variant lasso peptides related to siamycin I, sviceucin, or arcumycin that are suitable for the composition according to the present disclosure share a sequence identity of at least 50 %, preferably at least 60 %, preferably at least 70 %, preferably at least 80 %, preferably at least 90 %, with either SEQ ID No. 1, SEQ ID No. 2, or SEQ ID No. 3, respectively. Siamycin II (CAS number: 164802-69-1, corresponding to SEQ ID No. 4), aborycin (also known as RP-71955, PDB reference: 1RPB, corresponding to SEQ ID No. 5), specialicin (PDB reference: 6AK0, corresponding to SEQ ID No. 6), and humidimycin (corresponding to SEQ ID No. 7) are examples of variant lasso peptides sharing a sequence identity of at least 50 % with either SEQ ID No. 1, SEQ ID No. 2, or SEQ ID No. 3.

Preferentially, variant lasso peptides related to siamycin I, sviceucin, or arcumycin that are suitable for the composition according to the present disclosure share a sequence identity of at least 10 amino acids with either SEQ ID No. 1, SEQ ID No. 2, or SEQ ID No. 3, respectively. More preferentially, variant lasso peptides related to siamycin I, sviceucin, or arcumycin that are suitable for the composition according to the present disclosure share a sequence identity of at least 14 amino acids with either SEQ ID No. 1, SEQ ID No. 2, or SEQ ID No. 3, respectively. Even more preferentially, variant lasso peptides related to siamycin I, sviceucin, or arcumycin that are suitable for the composition according to the present disclosure share a sequence identity of at least 19 amino acids with either SEQ ID No. 1, SEQ ID No. 2, or SEQ ID No. 3, respectively.

The composition according to the present disclosure comprises an antibiotic. Preferentially, the antibiotic comprised in the composition according to the present disclosure is selected from the group consisting of vancomycin, teicoplanin, and bacitracin. For example, vancomycin can be acquired as vancomycin hydrochloride (CAS number: 1404-93-9) from Sigma-Aldrich (MO, USA) under reference V0045000. For example, bacitracin (CAS number: 1405-87-4) can be acquired from Sigma-Aldrich (MO, USA) under reference 11702. Preferentially, the antibiotic comprised in the composition according to the present disclosure is vancomycin.

In the context of the present disclosure the antibiotic can either be added to the nanoemulsion that already comprises the lasso peptide or added during the formation of the nanoemulsion that comprises the lasso peptide.

For example, the antibiotic can be prepared in the form of a pharmaceutically acceptable antibiotic nanoemulsion before the step of adding said antibiotic nanoemulsion to the pharmaceutically acceptable nanoemulsion already comprising the lasso peptide or during the preparation of the pharmaceutically acceptable nanoemulsion of said lasso peptide.

When the antibiotic is added to the nanoemulsion already comprising the lasso peptide, the addition of the antibiotic is preferentially performed extemporaneously, prior to the recovery of the composition.

When the antibiotic is added during the formation of the nanoemulsion that comprises the lasso peptide, the method for producing a composition comprising a lasso peptide formulated in a pharmaceutically acceptable nanoemulsion and an antibiotic comprises a step of forming a pharmaceutically acceptable nanoemulsion comprising both the lasso peptide and the antibiotic. In this embodiment of the present disclosure, when the antibiotic is hydrophilic (e.g., vancomycin) said antibiotic may be solubilized in the aqueous phase used in step d). On the other hand, when the antibiotic is hydrophobic (e.g., rifampicin) said antibiotic may be added during step a) to the other components to form the anhydrous phase.

In the composition according to the present disclosure, the lasso peptide is formulated in a pharmaceutically acceptable nanoemulsion. Nanoemulsions are a family of drug delivery systems that are known to the man with ordinary skill in the art of formulating therapeutic agents, as disclosed for example in documents Jaiswal M, Dudhe R, Sharma PK. Nanoemulsion: an advanced mode of drug delivery system. 3 Biotech. 2015 Apr;5(2):123-127 (Ref. No. 6) and Kumar M, Bishnoi RS, Shukla AK, Jain CP. Techniques for Formulation of Nanoemulsion Drug Delivery System: A Review. Prev Nutr Food Sci. 2019 Sep;24(3):225-234 (Ref. No. 7). Nanoemulsions suitable to perform the method for preparing a composition according to the present disclosure may be oil-in-water nanoemulsions. Preferentially, pharmaceutically acceptable oil-in-water nanoemulsions are obtained from ingredients regarded as Generally Recognized As Safe (GRAS) by the Food and Drug Administration (FDA) of the United States of America.

Preferentially, the lasso peptide is formulated in an oil-in-water nanoemulsion that is obtained at room temperature by a spontaneous emulsification process, by adding an aqueous phase to an anhydrous phase comprising the lasso peptide.

Preferentially, the nanoemulsion is obtained from:
an anhydrous phase comprising the following components:
   - the lasso peptide,
   - a surfactant,
   - an oil, and
   - a solubilizer;
and an aqueous phase.

The surfactant may be selected among those known to the person with ordinary skill in the art. Preferentially, the surfactant is chosen from anionic surfactants, non-ionic surfactants, cationic surfactants, amphoteric surfactants and extended surfactants containing a non-ionic spacer arm central extension and an ionic or nonionic polar group. For example, the surfactant can be a PEGlygated non-ionic surfactant such as PEG-15 hydroxystearate. Preferentially, the surfactant is PEG-15 hydroxystearate or a preparation thereof. Kolliphor^{®} HS 15 is a PEG-15 hydroxystearate preparation suitable for preparing the anhydrous phase of the composition according to the present disclosure. Kolliphor^{®} HS 15 can be acquired from BASF (Ludwigshafen, Germany).

The oil is either a natural oil or a synthetic oil, or a mixture thereof. When the oil is a natural oil, the oil may be a vegetable oil or an animal oil, or a mixture thereof. More preferentially, the oil is a medium-chain triglyceride or a preparation thereof. Particularly, the oil may be caprylic (C8) and capric (C10) acids, or a mix thereof. Labrafac^{®} lipophile WL 1349 is an example of synthetic oil. Labrafac^{®} lipophile WL 1349 is a medium-chain triglyceride preparation suitable for preparing the anhydrous phase of the composition according to the present disclosure. Labrafac^{®} lipophile WL 1349 can be acquired from Gattefossé (France).

The solubilizer may be selected among those known to the person with ordinary skill in the art. Preferentially, it is selected from glycerides, or can be a solvent such as dimethyl sulfoxide, ethanol or a derivate thereof. Preferentially, the solubilizer is diethylene glycol monoethyl ether or a preparation thereof. Transcutol^{®} HP is a diethylene glycol monoethyl ether preparation suitable for preparing the anhydrous phase of the composition according to the present disclosure. Transcutol^{®} HP can be acquired from Gattefossé (France).

For example, the anhydrous phase further comprises the following components:
- PEG-15 hydroxystearate as the surfactant,
- a medium-chain triglyceride as the oil, and
- diethylene glycol monoethyl ether as the solubilizer.

The anhydrous phase can comprise a co-surfactant. The co-surfactant may be selected as defined above for the surfactant. The co-surfactant and/or a solubilizer may be useful to optimize the stability and the drug-loading of the nanodroplets.

Preferentially, the composition of the anhydrous phase, without the lasso peptide may be as follows, in % by weight with regard to the total weight of the composition without the lasso peptide:
- From 45 % to 65 % by weight, preferably 55 % by weight of surfactant,
- From 30 % to 40 % by weight, preferably 35 % by weight of oil, and
- From 8 % to 12 % by weight, preferably 10 % by weight of solubilizer.

Preferentially, the composition of the anhydrous phase, without the lasso peptide may be as follows, in % by weight with regard to the total weight of the composition without the lasso peptide: 55 % by weight of surfactant, 35 % by weight of oil, and 10 % by weight of solubilizer.

Preferentially, the composition of the anhydrous phase, with the lasso peptide may be as follows, in % by weight with regard to the total weight of the composition with the lasso peptide:
- From 0.01 % to 2.5 % by weight, preferably 0.5 % by weight of lasso peptide,
- From 45 % to 65 % by weight, preferably 54.7 % by weight of surfactant,
- From 30 % to 40 % by weight, preferably 34.8 % by weight of oil, and
- From 8 % to 12 % by weight, preferably 10 % by weight of solubilizer.

Preferentially, the composition of the anhydrous phase, with the lasso peptide may be as follows, in % by weight with regard to the total weight of the composition with the lasso peptide: 0.5 % by weight of lasso peptide, 54.7 % by weight of surfactant, 34.8 % by weight of oil, and 10 % by weight of solubilizer.

The aqueous phase preferentially comprises or consists of water or an aqueous buffer. More preferentially, the aqueous phase comprises or consists of a phosphate buffer. Such an aqueous buffer may be useful to control the pH and the osmolarity of the preparation. More preferentially, the aqueous phase is in the form of an aqueous solution comprising a phosphate buffer with a phosphate concentration comprised between 55 mM and 65 mM. More preferentially, the aqueous phase is a phosphate buffer with a phosphate concentration of 60 mM.

An example of alternative nanemulsion that could be used to perform the method for preparing a composition according to the present disclosure has previously been described in E. Gu6, M. Since, S. Ropars, R. Herbinet, L. Le Pluart, A. Malzert-Fréon, Evaluation of the versatile character of a nanoemulsion formulation, International Journal of Pharmaceutics, Volume 498, Issues 1-2, 2016, Pages 49-65 (Ref. No. 8). Said alternative nanoemulsion was obtained from Kolliphor^{®} HS 15, Labrafac^{®} lipophile WL 1349 and Labrasol^{®}. Labrasol^{®} is a preparation of PEG-8 caprylic/capric glycerides. Labrasol^{®} can be acquired from Gattefossé (France).

### Methods for producing the compositions:

The present disclosure also relates to a method for producing a composition comprising a lasso peptide formulated in a pharmaceutically acceptable nanoemulsion and an antibiotic, said method comprising a step of adding said antibiotic to a pharmaceutically acceptable nanoemulsion already comprising the lasso peptide or during the preparation of a pharmaceutically acceptable nanoemulsion of said lasso peptide. For example, these nanoemulsion can be formulated by spontaneous emulsification or by other process such as microfluidic.

The document Séguy L, Groo AC, Goux D, Hennequin D, Malzert-Fréon A. Design of Non-Haemolytic Nanoemulsions for Intravenous Administration of Hydrophobic APIs. Pharmaceutics. 2020 Nov 25;12(12):1141 (Ref. No. 9) discloses methods to prepare a nanoemulsion. The inventors have determined a nanoemulsion prepared according to Seguy et al can be adapted to formulate a lasso peptide.

Preferentially, the method for producing a composition comprising a lasso peptide formulated in a pharmaceutically acceptable nanoemulsion and an antibiotic, comprises the following steps in the following order:
a) Mixing all together the lasso peptide, a surfactant, an oil, and a solubilizer to form an anhydrous phase,
b) Heating and homogenizing the anhydrous phase of step a),
c) Cooling the anhydrous mixture down of step b), under stirring,
d) Addition of the aqueous phase to the anhydrous phase of step c), under stirring,
e) Adding an antibiotic to the nanoemulsion obtained at step d) or during or before step d), and
f) Recovering the composition obtained at step e).

At step e), when the antibiotic is added to the nanoemulsion obtained at step d), said antibiotic may itself be formulated in a pharmaceutically acceptable nanoemulsion.

Preferentially, the goal temperature to be reached at the end of the cooling at step c) is 25 Celsius degrees (°C).

Preferentially, step b) of the method for producing a composition according to the present disclosure comprises three successive sub-steps b1), b2) and b3):
b1) heating the anhydrous phase under gentle stirring,
b2) homogenizing the anhydrous phase, and
b3) heating the homogenized anhydrous phase under gentle stirring.

Preferentially, the goal temperature to be reached at the end of the heating at step b), b1) and/or b3) is 70 °C.

Preferentially, the action of gentle stirring at step b1) and/or b3) is performed using magnetic stirring.

Preferentially, the action of gentle stirring at step b1) and/or b3) is performed using magnetic stirring with a rotation speed comprised between 200 rotations per minute (rpm) and 300 rpm. More preferentially, the action of gentle stirring at step b1) and/or b3) is performed using magnetic stirring with a rotation speed of 250 rpm.

Preferentially, the step of homogenizing the anhydrous phase is completed when the anhydrous phase reaches a visually homogeneous aspect.

Preferentially, the step d) of the method for producing a composition according to the present disclosure consists in three successive sub-steps d1), d2) and d3):
d1) increasing the stirring speed,
d2) consists in adding the aqueous phase,
d3) consists in maintaining stirring for 15 minutes at room temperature.

Preferentially, the nanoemulsion obtained after step d) exhibits a polydispersity index (PDI) of less than 0.2, when measured by dynamic light scattering (DLS).

Preferentially, the stirring speed to be reached at the end of step d1) is comprised between 700 rpm and 800 rpm, more preferably 750 rpm.

Preferentially, the aqueous phase added at step d2) is a phosphate buffer, more preferentially as defined above.

Preferentially, the method for producing a composition according to the present disclosure further comprises a step d'), performed after step d) and before step e), which comprises filtering the nanoemulsion, preferably through a 0.2 µm cellulose filter, preferably a regenerated cellulose filter. More preferentially, the filter is a 0.2 µm regenerated cellulose filter at step d') in the form of a syringe filter. Such 0.2 µm regenerated cellulose syringe filter can be acquired from Sartorius (Göttingen, Germany) under the brand name Minisart^{®} Syringe Filter.

When the antibiotic is added during the formation of the nanoemulsion that comprises the lasso peptide, the method for producing a composition comprising a lasso peptide formulated in a pharmaceutically acceptable nanoemulsion and an antibiotic comprises a step of forming a pharmaceutically acceptable nanoemulsion comprising both the lasso peptide and the antibiotic, before recovering the composition. In this embodiment, when the antibiotic is hydrophilic, the step of forming a pharmaceutically acceptable nanoemulsion preferentially involves mixing the antibiotic in the aqueous phase before step d) is performed. On the other hand, when is antibiotic is hydrophobic, the step of forming a pharmaceutically acceptable nanoemulsion preferentially involves mixing the antibiotic with the lasso peptide, the surfactant, the oil, and the solubilizer during step a) to form the anhydrous phase.

### Uses of the compositions:

The present disclosure also relates to a composition as defined above for its use as a medicine, to a use of a composition as defined above for treating an infection, and to a method for treating an infection by a Gram-positive bacterium comprising the step of administering to a patient a composition as defined above.

Preferentially, the composition according to the present disclosure is used as a medicine for a human patient or an animal.

Preferentially, the Gram-positive bacterium exhibits resistance to the antibiotic, when said antibiotic is administered alone.

Preferentially, the Gram-positive bacterium harbors a *van* operon comprising the sensor kinase VanS. The sensor kinase VanS has previously been studied in document Lockey C, Edwards RJ, Roper DI, Dixon AM. The Extracellular Domain of Two-component System Sensor Kinase VanS from Streptomyces coelicolor Binds Vancomycin at a Newly Identified Binding Site. Sci Rep. 2020 Mar 31;10(1):5727 (Ref. No. 10). Preferentially, the *van* operon may be the *vanA, vanB, vanC, vanD, vanE, vanG, vanL, vanM,* or *vanN* type, comprising respectively the sensor kinase VanS, VanSs, VanSc, VanS_{D}, VanS_{E}, VanSc, VanS_{L}, VanS_{M}, VanS_{N}.

Preferentially, the Gram-positive bacterium belongs to the *Enterococcus* genus, the *Staphylococcus* genus, and the *Clostridioides* genus. More preferentially, the Gram-positive bacterium is selected from the group consisting of *Enterococcus faecalis, Enterococcus faecium, Staphylococcus aureus* and *Clostridioides difficile.*

The composition, the lasso peptide, the antibiotic, the surfactant, the oil and the solubilizer may be as defined above.

The method for treating an infection according to the present disclosure comprises the step of administering to a patient a composition as defined above.

The composition according to the present disclosure can be administered to the patient by any suitable route of administration to reach the organs or tissues of the patient where the Gram-positive bacterium can enter, has entered, has transited through or has multiplied in the patient's body. For example, the composition of the present disclosure may be administered in a patient or animal to be treated, for example topically, orally, intravenously, or intraperitoneally. Preferably, the composition according to the present disclosure is administered orally or intravenously.

Preferentially, the composition according to the present disclosure is administered to the patient in an amount corresponding to an administered quantity of lasso peptide comprised between 1 to 50 mg of lasso peptide per kg of patient weight. More preferentially, the composition according to the present disclosure is administered to the patient in an amount corresponding to an administered quantity of lasso peptide comprised between 2 to 20 mg of lasso peptide per kg of patient weight. Even more preferentially, the composition according to the present disclosure is administered to the patient in an amount corresponding to an administered quantity of lasso peptide comprised between 3 to 10 mg of lasso peptide per kg of patient weight. Preferably, the composition according to the present disclosure is administered to the patient in an amount corresponding to an administered quantity of lasso peptide of about 5 mg of lasso peptide per kg of patient weight.

Preferentially, the composition according to the present disclosure is administered to the patient in an amount corresponding to an administered quantity of antibiotic comprised between 1 to 50 mg of antibiotic per kg of patient weight. More preferentially, the composition according to the present disclosure is administered to the patient in an amount corresponding to an administered quantity of antibiotic comprised between 2 to 20 mg of antibiotic per kg of patient weight. Even more preferentially, the composition according to the present disclosure is administered to the patient in an amount corresponding to an administered quantity of antibiotic comprised between 3 to 10 mg of antibiotic per kg of patient weight. Preferably, the composition according to the present disclosure is administered to the patient in an amount corresponding to an administered quantity of antibiotic of about 5 mg of antibiotic per kg of patient weight.

### Kit for obtaining a composition according to the present disclosure:

The present disclosure also relates to a kit for obtaining the composition according to any of claims 1 to 9 wherein said kit comprises the following components:
- A first component comprising a pharmaceutically acceptable nanoemulsion in which a lasso peptide is formulated, and
- A second component comprising an antibiotic; and
wherein the composition is obtained by adding and mixing extemporaneously the second component to the first component.

### Brief description of the figures

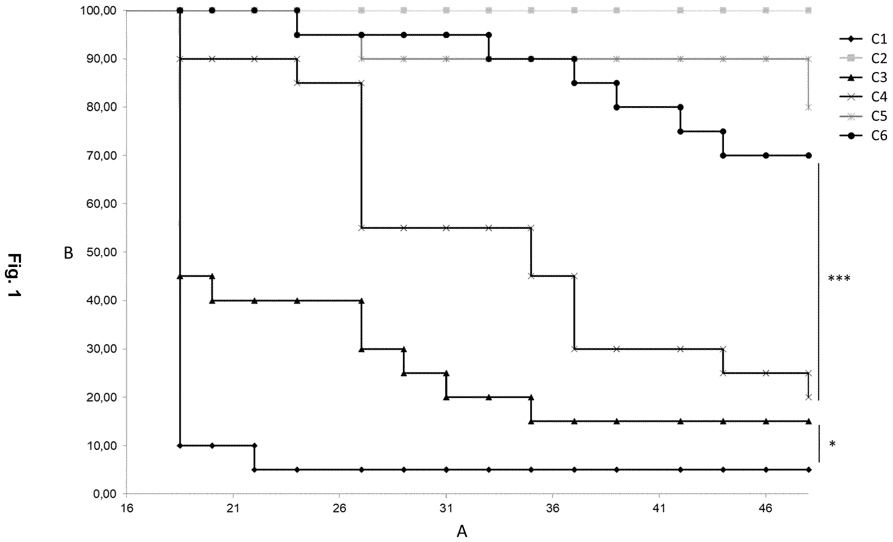

*Figure 1**:* a Caterpillar survival, Fig. 1 is a graph showing the survival rate of *Galleria mellonella* larvae after they received an inoculum and then received a treatment.

### Examples

### Example 1: Preparation of a composition according to the present disclosure wherein siamycin I is formulated in a nanoemulsion to which vancomycin is added

2.2 ml of a composition comprising nanoemulsion-formulated siamycin I with vancomycin added to the siamycin I-loaded nanoemulsion were prepared as follows:
- Mixing 1.2 mg of siamycin I with 132.5 mg of Kolliphor^{®} HS 15 (Sigma-Aldrich), 84.2 mg of Labrafac^{®} lipophile WL 1349 (Gattefossé) and 24.2 mg of Transcutol^{®} HP (Gattefossé) to form an anhydrous phase,
- Heating the anhydrous phase at 70 °C under gentle magnetic stirring (250 rpm),
- Sonication of the anhydrous mixture for a few minutes,
- Then, the anhydrous phase was heated at 70 °C under gentle magnetic stirring,
- Then, the anhydrous phase was cooled down at 25 °C,
- Increasing magnetic stirring from 250 rpm to 750 rpm,
- Adding 2 ml of phosphate buffer with a phosphate concentration of 60 mM (25 °C),
- Maintaining stirring for 15 minutes at room temperature,
- Filtering the obtained nanoemulsion through a 0.2 µm regenerated cellulose syringe filter (Minisart^{®} Syringe Filter, Sartorius, Göettingen, Germany),
- Adding vancomycin by extemporaneous mixing with formulated siamycin I-loaded nanoemulsion,
- Recovering the obtained siamycin I-loaded nanoemulsion with vancomycin.

### Example 2: Preparation of a control nanoemulsion

A control nanoemulsion was prepared as follows:
- Mixing 133.1 mg of Kolliphor^{®} HS 15 (Sigma-Aldrich), 84.7 mg of Labrafac^{®} lipophile WL 1349 (Gattefossé) and 24.2 mg of Transcutol^{®} HP (Gattefossé) to form an anhydrous phase,
- Heating the anhydrous phase at 70 °C under gentle magnetic stirring (250 rpm),
- Sonication of the anhydrous mixture for a few minutes,
- Then, the anhydrous phase was heated at 70 °C under gentle magnetic stirring,
- Then, the anhydrous phase was cooled down at 25 °C,
- Increasing magnetic stirring from 250 rpm to 750 rpm,
- Adding 2 ml of phosphate buffer with a phosphate concentration of 60 mM (25 °C),
- Maintaining stirring for 15 minutes at room temperature,
- Filtration of the obtained nanoemulsion through a 0.2 µm sterile regenerated cellulose syringe filter (Minisart^{®} Syringe Filter, Sartorius, Göttingen, 20 Germany), and
- Recovering the obtained control nanoemulsion.

### Example 3: Preparation of a control nanoemulsion to which vancomycin is added

A nanoemulsion to which vancomycin was added was prepared from a control nanoemulsion prepared as described in example 2, by extemporaneously mixing vancomycin to the control nanoemulsion before recovering it.

### Example 4: Preparation of a composition comprising nanoemulsion-formulated siamycin I without vancomycin

A nanoemulsion comprising siamycin I was prepared as according to example 1 except for the fact that no vancomycin was added before it was recovered.

### Example 5: In vivo trial in caterpillar (Galleria mellonella) larvae

In-house reared 5- to 7-weeks old *Galleria mellonella* larvae were infected subcutaneously using a syringe pump (KD scientific) with the V583 strain of *Enterococcus faecalis* (in experimental conditions C1, C3, C4, and C6), or with the V583Δ*vanB* strain of *Enterococcus faecalis* (in experimental condition C5), or with physiological water (in experimental condition C2). Bacteria were collected from overnight cultures by centrifugation, then washed and resuspended in physiological water in order to inoculate 10^6 CFU into each larva or the equivalent volume of physiological water as control. Twenty insects were used per condition. The V583 strain of *Enterococcus faecalis* (also noted *E*. *faecalis* V583) exhibits VanB resistance to vancomycin.

The V583Δ*vanB* strain of *Enterococcus faecalis* (also noted *E*. *faecalis* V583Δ*vanB*) is a strain derived from E. *faecalis* V583 that has lost its VanB resistance to vancomycin.

At 1.5 and 24 hours post-infection, larvae were injected either with 10 µl of a composition according to Example 1 (with a siamycin I concentration of 392 µg/ml and a vancomycin concentration of 100 µg/ml), or with 10 µl of a control nanoemulsion according to Example 2, or with 10 µl of a nanoemulsion to which vancomycin was added as according to Example 3 (with a vancomycin concentration of 100 µg/ml), or with 10 µl of a nanoemulsion comprising siamycin I as according to Example 4 (with a siamycin I concentration of 392 µg/ml).

The death of the larvae was then monitored between 1.5 and 48 hours post-infection. Data and statistical analysis were performed using the Kaplan-Meier R package and the log rank test, respectively. The results of this monitoring were reported in Figure 1.

In Figure 1 (Fig. 1):
- The A axis represents post-infection time, expressed in hours.
- The B axis represents the survival rate of *Galleria mellonella* larvae, expressed in percentage with regard to the number of larvae tested for the same experimental condition.
- The experimental conditions used in the present example are described in the Table No. 3, as follows:

**Table No. 3: Description of the experimental conditions of Example 5**

| **Experimental condition** | **Inoculum** | **Treatment** |
|---|---|---|
| C1 | *E. faecalis* V583 | Nanoemulsion of Example 2 |
| C2 | Physiological water | Nanoemulsion of Example 4 |
| C3 | *E. faecalis* V583 | Nanoemulsion of Example 4 |
| C4 | *E. faecalis* V583 | Nanoemulsion of Example 3 |
| C5 | *E. faecalis* V583Δ*vanB* | Nanoemulsion of Example 3 |
| C6 | *E. faecalis* V583 | Nanoemulsion of Example 1 |

20 insects were used for each experimental condition.

### Results

Experimental condition C2 shows that in the absence of *Enterococcus faecalis* in the inoculum, all *Galleria mellonella* larvae survived at least 46 hours after the time of inoculation.

Experimental condition C1 shows that approximately 95 % of the *Galleria mellonella* larvae that received an inoculum containing the V583 strain of *Enterococcus faecalis* and were treated with a control nanoemulsion died within the first 26 hours post-infection.

Experimental condition C3 shows that approximately 85 % of the *Galleria mellonella* larvae that received an inoculum containing the V583 strain of *Enterococcus faecalis* and were treated with a nanoemulsion containing siamycin I but no vancomycin died within the first 36 hours post-infection.

Experimental condition C4 shows that approximately 80 % of the *Galleria mellonella* larvae that received an inoculum containing the V583 strain of *Enterococcus faecalis* and were treated with a control nanoemulsion with vancomycin died within the first 46 hours post-infection.

Experimental condition C5 shows that approximately 10 % of the *Galleria mellonella* larvae that received an inoculum containing the V583Δ*vanB* strain of *Enterococcus faecalis* and were treated with a control nanoemulsion with vancomycin died within the first 46 hours post-infection.

Experimental condition C6 shows that approximately 30 % of the *Galleria mellonella* larvae that received an inoculum containing the V583 strain of *Enterococcus faecalis* and were treated with a nanoemulsion containing siamycin I with vancomycin died within the first 46 hours post-infection.

At 48 hours post-infection, the survival rates obtained for experimental conditions C5 and C6 did not differ significantly from one another.

At 48 hours post-infection, the survival rates obtained for experimental conditions C5 and C6 did differ significantly from the survival rates obtained for experimental conditions C1, C3, and C4.

### Conclusions

The results described above suggest that the use of a composition according to the present disclosure (as defined in Example 1) to treat an infection of *Galleria mellonella* larvae infected by vancomycin-resistant *E*. *faecalis* V583 provides similar results than the treatment with vancomycin of *Galleria mellonella* larvae infected by a *E. faecalis* strain that is not resistant to vancomycin.

### Example 6: In vitro evaluation of bacterial growth

### Material and method

An evaluation of bacterial growth was performed *in vitro* to assess the bacterial response to either vancomycin or bacitracin, in the absence or in the presence of either sviceucin or siamycin I.

Bacterial strains used in this evaluation are *E*. *faecalis* V583 (exhibiting VanB resistance), *E. faecium* Bi21 (exhibiting VanA resistance), *E. faecium* Bi22 (exhibiting VanA resistance).

Each strain was cultured at 37 °C in M17 medium (Terzaghi and Sandine 1975) supplemented with 0.5 % (weight/volume) glucose (GM17 medium). Overnight cultures are grown in GM17 broth and are used to inoculate the cultures for MIC measurement.

Vancomycin was purchased from Sigma-Aldrich (MO, USA).

Bacitracin was purchased from Sigma-Aldrich (MO, USA).

Lyophilized lasso peptides were solubilized first at 800 µM in a 1:1 solution of sterilized pure water and methanol. This stock is used for subsequent dilutions in GM17.

The measurement of the minimal inhibitory concentration (MIC) of antibiotic (either vancomycin or bacitracin) was determined by the microdilution method in accordance with the guidelines of the Clinical and Laboratory Standards Institute (CLSI 2012), with GM17 medium used as a growth medium. Subsequent 2-fold serial dilutions of the antibiotic (either vancomycin or bacitracin) were performed to obtain concentrations ranging from 0.5 to 512 µg/mL.

The lasso peptides (either siamycin I or sviceucin) were added for the relevant conditions at a final concentration of 10 µM, 5 µM or 2.5 µM. Bacterial strains from overnight cultures were added with an inoculum of 5 x 10^5 CFU/mL. The MIC corresponds to the lowest concentration in the wells where no growth is observed.

### Results

The results that were obtained are presented in Table No. 4. The values of the minimal inhibitory concentration (MIC) are expressed in µg/mL.

### Table No. 4: Results of the in vitro evaluation of bacterial growth

This table presents the results of the *in vitro* evaluation of the bacterial response to either vancomycin or bacitracin, in the absence or in the presence of either sviceucin or siamycin I.

**Table No. 4: Results of the in vitro evaluation of bacterial growth**

| **Conditions** | **Growth in the presence of the lasso peptide alone** | **Vancomycin MIC (VanB-resistant strain)** | **Vancomycin MIC (VanA-resistant strains)** | **Bacitracin MIC** |
|---|---|---|---|---|
| Without lasso peptide | Normal growth | 32 | 256 | 512 |
| Sviceucin (10 µM) | Growth slowed down but not inhibited | 2 | 128 | 16 |
| Siamycin I (2.5 µM) | Growth slowed down but not inhibited | 2 | 2 | 16 |
| Siamycin I (5 µM) | Growth inhibited | 0 | 0 | 0 |

### Conclusions

Sviceucin at 10 µM combined with vancomycin reverses vancomycin resistance in VanB-type vancomycin resistant Gram-positive bacteria belonging to the Enterococcus genus.

Sviceucin at 10 µM combined with bacitracin reverses bacitracin resistance in bacitracin resistant Gram-positive bacteria belonging to the Enterococcus genus.

Siamycin I at 2.5 µM combined with vancomycin reverses vancomycin resistance in VanA- and VanB-type vancomycin resistant Gram-positive bacteria belonging to the Enterococcus genus.

Siamycin I at 2.5 µM combined with bacitracin reverses bacitracin resistance in bacitracin resistant Gram-positive bacteria belonging to the Enterococcus genus.

Siamycin I at 5 µM exhibits a growth inhibition activity in VanA- and VanB-type vancomycin resistant Gram-positive bacteria belonging to the Enterococcus genus and bacitracin resistant Gram-positive bacteria belonging to the Enterococcus genus. In contrast, Sviceucin at 10 µM does not affect growth.

### Example 7: Preparation of a composition according to the present disclosure wherein vancomycin and siamycin I are formulated in a pharmaceutically acceptable nanoemulsion

A composition according to the present disclosure, wherein siamycin I is formulated in a pharmaceutically acceptable nanoemulsion and wherein vancomycin is added during the step of forming the siamycin I-loaded nanoemulsion may be prepared by the same method than the one disclosed at example 1, except for the fact that vancomycin is added to the phosphate buffer prior to its mixing with the anhydrous phase to form the siamycin I-loaded nanoemulsion.

### List of references

**Ref. No. 1:** WHO publishes list of bacteria for which new antibiotics are urgently needed. This article can be accessed with this URL address: https://www.who.int/news/item/27-02-2017-who-publishes-list-of-bacteria-for which-new-antibiotics-are-urgently-needed
**Ref. No. 2:** McCallum N, Karauzum H, Getzmann R, Bischoff M, Majcherczyk P, Berger-Bächi B, Landmann R. In vivo survival of teicoplanin-resistant Staphylococcus aureus and fitness cost of teicoplanin resistance. Antimicrob Agents Chemother. 2006 Jul;50(7):2352-60. doi: 10.1128/AAC.00073-06. PMID: 16801412; PMCID: PMC1489778.
**Ref. No. 3:** Kawalec M, Gniadkowski M, Kedzierska J, Skotnicki A, Fiett J, Hryniewicz W. Selection of a teicoplanin-resistant Enterococcus faecium mutant during an outbreak caused by vancomycin-resistant enterococci with the vanB phenotype. J Clin Microbiol. 2001 Dec;39(12):4274-82. doi: 10.1128/JCM.39.12.4274-4282.2001. PMID: 11724832; PMCID: PMC88536.
**Ref. No. 4:** Hegemann JD, Zimmermann M, Xie X, Marahiel MA. Lasso peptides: an intriguing class of bacterial natural products. Acc Chem Res. 2015 Jul 21;48(7):1909-19. doi: 10.1021/acs.accounts.5b00156. Epub 2015 Jun 16. PMID: 26079760.
**Ref. No. 5:** Li Y, Ducasse R, Zirah S, Blond A, Goulard C, Lescop E, Giraud C, Hartke A, Guittet E, Pernodet JL, Rebuffat S. Characterization of Sviceucin from Streptomyces Provides Insight into Enzyme Exchangeability and Disulfide Bond Formation in Lasso Peptides. ACS Chem Biol. 2015 Nov 20;10(11):2641-9. doi: 10.1021/acschembio.5b00584. Epub 2015 Sep 21. PMID: 26343290.
**Ref. No. 6:** Jaiswal M, Dudhe R, Sharma PK. Nanoemulsion: an advanced mode of drug delivery system. 3 Biotech. 2015 Apr;5(2):123-127. doi: 10.1007/s13205-014-0214-0. Epub 2014 Apr 8. PMID: 28324579; PMCID: PMC4362737.
**Ref. No. 7:** Kumar M, Bishnoi RS, Shukla AK, Jain CP. Techniques for Formulation of Nanoemulsion Drug Delivery System: A Review. Prev Nutr Food Sci. 2019 Sep;24(3):225-234. doi: 10.3746/pnf.2019.24.3.225. Epub 2019 Sep 30. PMID: 31608247; PMCID: PMC6779084.
**Ref. No. 8:** E. Gu6, M. Since, S. Ropars, R. Herbinet, L. Le Pluart, A. Malzert-Fréon, Evaluation of the versatile character of a nanoemulsion formulation, International Journal of Pharmaceutics, Volume 498, Issues 1-2, 2016, Pages 49-65, ISSN 0378-5173, doi: 10.1016/j.ijpharm.2015.12.010.
**Ref. No. 9:** Séguy L, Groo AC, Goux D, Hennequin D, Malzert-Fréon A. Design of Non-Haemolytic Nanoemulsions for Intravenous Administration of Hydrophobic APIs. Pharmaceutics. 2020 Nov 25;12(12):1141. doi: 10.3390/pharmaceutics12121141. PMID: 33255606; PMCID: PMC7760703.
**Ref. No. 10:** Lockey C, Edwards RJ, Roper DI, Dixon AM. The Extracellular Domain of Two-component System Sensor Kinase VanS from Streptomyces coelicolor Binds Vancomycin at a Newly Identified Binding Site. Sci Rep. 2020 Mar 31;10(1):5727. doi: 10.1038/s41598-020-62557-z. PMID: 32235931; PMCID: PMC7109055.

## Claims

1. A composition comprising a lasso peptide formulated in a pharmaceutically acceptable nanoemulsion and an antibiotic, wherein the composition is obtained by a process comprising a step of adding said antibiotic to a pharmaceutically acceptable nanoemulsion already comprising the lasso peptide or during the preparation of a pharmaceutically acceptable nanoemulsion of said lasso peptide.

2. A composition according to claim 1, wherein the antibiotic is prepared in the form of a pharmaceutically acceptable antibiotic nanoemulsion before the step of adding said antibiotic nanoemulsion to the pharmaceutically acceptable nanoemulsion already comprising the lasso peptide or during the preparation of the pharmaceutically acceptable nanoemulsion of said lasso peptide.

3. A composition according to claim 1, wherein the lasso peptide is a type I lasso peptide.

4. A composition according to claim 2, wherein the lasso peptide is selected from the group consisting of siamycin I, sviceucin, arcumycin, siamycin II, aborycin, specialicin, humidimycin, variants of siamycin I, variants of sviceucin, and variants of arcumycin.

5. A composition according to any of claims 1 to 4, wherein the antibiotic is selected from the group consisting of vancomycin, teicoplanin and bacitracin.

6. A composition according to any of claims 1 to 5, wherein the nanoemulsion is obtained from:
an anhydrous phase comprising the following components:
- the lasso peptide,
- a surfactant,
- an oil, and
- a solubilizer;
and an aqueous phase.

7. A composition according to claim 6, wherein the anhydrous phase further comprises the following components:
- PEG-15 hydroxystearate as the surfactant,
- a medium-chain triglyceride as the oil, and
- diethylene glycol monoethyl ether as the solubilizer.

8. A composition according to claim 6 or 7, wherein the aqueous phase is a phosphate buffer with a phosphate concentration comprised between 55 mM and 65 mM.

9. A composition according to any of claims 5 to 7, wherein the components for the anhydrous phase are in the following proportions by weight of the anhydrous phase:
- 0.5 % of the lasso peptide,
- 54.7 % of PEG-15 hydroxystearate,
- 34.8 % of a medium-chain triglyceride, and
- 10 % of diethylene glycol monoethyl ether.

10. A method for producing a composition as defined in claim 1, comprising a step of forming a pharmaceutically acceptable nanoemulsion comprising the lasso peptide.

11. The method of claim 10, comprising the following steps in the following order:
a) Mixing all together the lasso peptide, a surfactant, an oil, and a solubilizer to form an anhydrous phase,
b) Heating and homogenizing the anhydrous phase of step a),
c) Cooling the anhydrous mixture down of step b), under stirring,
d) Addition of the aqueous phase to the anhydrous phase of step c), under stirring,
e) Adding an antibiotic to the nanoemulsion obtained at step d) or during or before step d), and
f) Recovering the composition obtained at step e).

12. A composition according to any of claims 1 to 9 for use as a medicine.

13. A composition for use according to claim 12, wherein said medicine is for the treatment of an infection by a Gram-positive bacterium.

14. A composition for use according to claim 13, wherein the Gram-positive bacterium exhibits resistance to the antibiotic alone.

15. A composition for use according to claim 13 or 14, wherein the Gram-positive bacterium belongs to the *Enterococcus* genus, the *Staphylococcus* genus, and the *Clostridioides* genus.

16. A composition for use according to claim 15, wherein the Gram-positive bacterium is selected from the group consisting of *Enterococcus faecalis, Enterococcus faecium, Staphylococcus aureus* and *Clostridioides difficile.*

17. A kit for obtaining the composition according to any of claims 1 to 9 wherein said kit comprises the following components:
- A first component comprising a pharmaceutically acceptable nanoemulsion in which a lasso peptide is formulated, and
- A second component comprising an antibiotic; and
wherein the composition is obtained by adding and mixing extemporaneously the second component to the first component.
